# DEMANDE DE BREVET EUROPEEN

(11) **EP 1 464 317 A1**
(43) Date de publication de la demande: **06.10.2004**
(21) Numéro de dépôt: 04290610.7
(22) Date de dépôt: 23.12.1998
(51) Int. Cl.: A61K 7/06, A61K 7/48

(54) **Composition cosmétique à base de polyuréthannes associatifs et de polymères non ioniques à chaines grasses**

(30) Priorité: 13.02.1998 FR 9801777
(62) Demande divisionnaire de: 98963625.3
(71) Demandeur: L'OREAL, 75008 Paris (FR)
(72) Inventeur: Dupuis, Christine, 75018 Paris (FR)
(74) Mandataire: Bourdeau, Françoise

(57) **Abrégé**

La présente invention concerne des compositions cosmétiques contenant, dans un milieu cosmétiquement acceptable, en tant que système épaississant,
(A) au moins un polyuréthanne associatif non ionique et
(B) au moins un polymère non ionique comportant au moins un motif à chaîne grasse.

## Description

La présente invention concerne des compositions cosmétiques contenant un nouveau système épaississant de milieux aqueux à base de polyuréthannes associatifs et de polymères non ioniques à chaînes grasses, ainsi que leur utilisation en tant que gels coiffants ou gels de soin capillaires non rincés.

L'épaississement et/ou la gélification des milieux aqueux par des polymères est depuis longtemps un important de sujet de recherche cosmétique. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Une approche intéressante a consisté à utiliser comme épaississants des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.

De tels polymères capables de s'associer réversiblement entre eux ou avec d'autres molécules sont appelés "polymères associatifs". Les forces d'interactions en jeu peuvent être de nature très différente par exemple de nature électrostatique, de type liaisons hydrogène ou des interactions hydrophobes.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

Il est connu de préparer des compositions capillaires sous forme de gel utilisant, comme système épaississant, de tels polymères amphiphiles associatifs, en conjonction avec des agents tensioactifs. On pense que les propriétés rhéologiques intéressantes des gels ainsi obtenus sont dues à la formation de micelles mixtes contenant les agents tensioactifs et les parties hydrophobes des polymères amphiphiles, ces micelles constituant une multitude de points de réticulation physique.

Cependant, ces compositions à base de polymères associatifs et d'agents tensioactifs n'ont pas toujours les propriétés cosmétiques souhaitées. Ainsi, la présence d'agents tensioactifs, même en faibles quantités, peut modifier de façon indésirable les propriétés cosmétiques desdites compositions, telles que les propriétés d'application ou de toucher après séchage. Par ailleurs, notamment dans le domaine des gels de coiffage ou de soin non rincés, il est important de pouvoir répartir uniformément le produit sur l'ensemble de la chevelure de manière à éviter les surcharges et les défauts cosmétiques qui en résultent.

On a découvert à présent qu'il était possible d'obtenir un bon effet épaississant, voire gélifiant, et des propriétés cosmétiques intéressantes en associant des polyuréthannes associatifs non ioniques à des polymères non ioniques comportant au moins un motif à chaîne grasse.

Le gel obtenu par l'association de ces deux types de polymères a une texture très fondante et est agréable à appliquer. Le toucher final sur cheveux séchés est plus agréable et moins chargé. Le gel a par ailleurs un excellent pouvoir coiffant.

Un objet de la présente invention est donc une composition cosmétique comprenant au moins un polyuréthanne non ionique associatif en combinaison avec au moins un polymère non ionique comportant au moins un motif à chaîne grasse.

Un autre objet de la présente invention est l'utilisation de la combinaison d'au moins un polyuréthanne non ionique associatif et d'au moins un polymère non ionique comportant au moins un motif à chaîne grasse en tant que système épaississant pour des compositions cosmétiques.

Un troisième objet de l'invention est un procédé de traitement cosmétique des cheveux utilisant une composition cosmétique obtenue par association d'au moins un polyuréthanne associatif non ionique et d'au moins un polymère non ionique comportant au moins un motif à chaîne grasse.

D'autres objets apparaîtront à la lecture de la description et des exemples qui suivront.

Les compositions cosmétiques conformes à l'invention sont essentiellement caractérisées par le fait qu'elles contiennent, dans un milieu cosmétiquement acceptable,
(A) au moins un polyuréthanne associatif non ionique correspondant à la formule générale 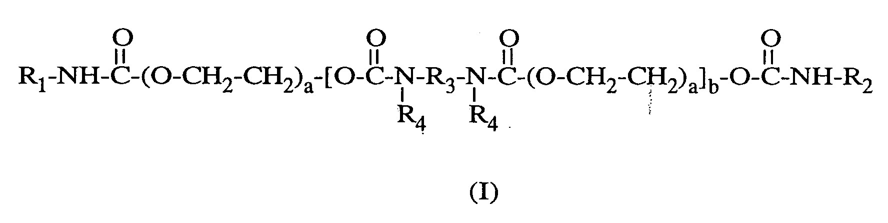 dans laquelle
   au moins un des résidus R₁ et R₂ représente un groupe alkyle supérieur en C₈₋₁₈ et l'autre, le cas échéant, un groupe alkyle inférieur en C₁₋₆,
   R₃ représente un radical hydrocarboné en C₄₋₃₆, de préférence en C₆₋₁₀,
   R₄ représente un atome d'hydrogène ou un radical alkyle en C₁₋₆, de préférence un atome d'hydrogène,
   a varie indépendamment de 90 à 600, et
   b vaut de 1 à 4, et
(B) au moins un polymère non ionique comportant au moins un motif à chaîne grasse et qui est différent du polyuréthanne de formule (I).

On entend par groupe alkyle inférieur en C₁₋₆, selon l'invention, un groupe alkyle à chaîne linéaire ou ramifiée comportant de 1 à 6 atomes de carbone, tel que les radicaux méthyle, éthyle, *n*-propyle, *n*-butyle, *n*-pentyle et *n*-hexyle ainsi que les isomères ramifiés correspondants.

Conformément à l'invention, les groupes alkyle supérieurs en C₈₋₁₈ désignent des groupes alkyle à chaîne linéaire ou ramifiée comportant de 8 à 18 atomes de carbone, tels que les radicaux octyle, nonyle, décyle, undécyle, dodécyle, tridécyle, tétradécyle, pentadécyle, hexadécyle, heptadécyle et octadécyle.

Dans un mode de réalisation préféré, un seul des radicaux alkyle R₁ et R₂ en α―Ω du polymère de formule (I) représente un groupe alkyle supérieur en C₈₋₁₈ et l'autre un groupe alkyle inférieur en C₁₋₆.

Un polyuréthanne associatif particulièrement recommandé de la présente invention est celui dans lequel un des groupes R₁ et R₂ représente un radical octadécyle et l'autre un groupe méthyle.

Les polyuréthannes associatifs utilisés dans les compositions de la présente invention sont utilisés sous forme de solution ou suspension aqueuse contenant éventuellement une certaine quantité d'amidon soluble. Cet amidon peut être n'importe quel amidon extrait de sources naturelles, tel que l'amidon de blé, de maïs, de riz, de pomme de terre etc., et qui a été modifié par voie chimique, enzymatique ou microbiologique de manière à être soluble dans l'eau.

Un polymère préféré est commercialisé par la Société Rohm & Haas sous la dénomination ACRYSOL 46. Il s'agit d'un polyuréthanne obtenu par condensation d'hexaméthylènediisocyanate et de polyéthylèneglycol, et portant à ses extrémités respectivement en moyenne un résidu méthyle et un résidu octadécyle. Ce polymère se présente sous forme d'une solution aqueuse à 15 % en poids de matière active polyuréthanne contenant, en plus, de 3 - 5 % d'une matrice d'amidon modifié par voie enzymatique.

Les polymères non ioniques comportant au moins une chaîne grasse selon la présente invention, utilisés comme composant (B), sont choisis de préférence parmi :
(2) les hydroxypropylguars modifiés par des groupes comportant au moins une chaîne grasse en C₈₋₂₂ tels que le produit ESAFLOR® HM 22 commercialisé par la société LAMBERTI, et les produits MIRACARE® XC95-3 et RE205-1 vendus par la société RHONE POULENC;
(3) les copolymères de vinylpyrrolidone et de monomères hydrophobes à chaîne grasse en C₈₋₂₂, comme par exemple les produits ANTARON® V216, ANTARON® V220, GANEX® V261 et GANEX®V220 commercialisés par la société I.S.P.;
(4) les copolymères de méthacrylates ou d'acrylates d'alkyle en C₁₋₆ et de monomères amphiphiles comportant au moins une chaîne grasse en C₈₋₂₂ tels que par exemple le copolymère méthacrylate de méthyle/acrylate de stéaryle oxyéthyléné vendu par la société GOLDSCHMIDT sous la dénomination ANTIL® 208;
(6) les polyuréthannes comportant au moins un motif à chaîne grasse différents des polyuréthannes de formule (I).

Selon l'invention, les polyuréthannes associatifs et les polymères non ioniques à chaînes grasses sont utilisés en des quantités suffisantes pour obtenir un épaississement ou une gélification satisfaisante du milieu aqueux.

On recommande notamment une quantité de polyuréthannes associatifs comprise entre 0,1 et 10 % en poids et, de préférence, entre 0,5 et 5 % en poids exprimé en matière active et rapportée au poids total de la composition.

Dans les compositions de la présente invention, les polymères non ioniques comportant au moins un motif à chaîne grasse sont présents à raison de 0,01 à 10 % en poids, de préférence à raison de 0,1 à 5 % en poids de matière active par rapport au poids total de la composition.

Dans la présente invention , le rapport dudit polyuréthanne associatif non ionique (A) de formule (I) audit polymère non ionique comportant au moins un motif à chaîne grasse (B) est compris de préférence dans l'intervalle allant de 90/10 à 10/90.

Le milieu cosmétiquement acceptable est constitué de préférence d'eau et peut contenir en outre des solvants cosmétiquement acceptables, par exemple des monoalcools inférieurs tels que l'éthanol ou l'isopropanol, des glycols tels que le diéthylèneglycol, des éthers de glycols tels que les alkyléthers d'éthylèneglycol ou de diéthylèneglycol, ou encore des esters d'acides gras, tous ces solvants étant utilisés seuls ou sous forme de mélange.

Les gels coiffants ou de soins peuvent contenir en outre un ou plusieurs additifs utilisés habituellement dans de telles compositions capillaires. On peut citer à titre d'exemple les parfums, colorants, conservateurs, filtres solaires, vitamines, agents régulateurs de pH etc. Il est bien entendu que le choix de ces composés doit tenir compte d'éventuelles interactions avec le système épaississant. L'homme du métier veillera à ce que l'adjonction de ces additifs n'ait pas d'influence défavorable sur les propriétés avantageuses des compositions obtenues grâce à la présente invention.

Un procédé de traitement cosmétique des cheveux préféré, selon l'invention, consiste à appliquer et à répartir de façon homogène les compositions décrites ci-dessus sur les cheveux et à sécher les cheveux ainsi traités sans les rincer.

Les exemples suivants sont destinés à illustrer l'invention sans pour autant présenter un caractère limitatif.

### Exemple 2

On a préparé un gel ayant la composition suivante :

| | |
|---|---|
| ACRYSOL46 | 3 % de matière active |
| AMERCELLPOLYMERHM 1500* | 2 % de matière active |
| eau déminéralisée | q. s. p. 100 g |

| | |
|---|---|
| * Le polymère AMERCELL POLYMER HM 1500 est une nonoxynyl-hydroxyéthylcellulose commercialisée par la société AMERCHOL. | |

### Exemple 3

On a préparé un gel ayant la composition suivante :

| | |
|---|---|
| ACRYSOL46 | 4 % de matière active |
| BERMODOLPUR2130* | 3 % de matière active |
| eau déminéralisée | q. s. p. 100 g |

| | |
|---|---|
| *Le polymère BERMODOL PUR 2130 (commericalisé par la société BEROL NOBEL) est un polyuréthanne modifié (en solution aqueuse à 25 %) | |

Il est bien entendu que la description qui précède n'a été donnée qu'à titre purement illustratif et non limitatif et que des variantes ou des modifications peuvent y être apportées dans le cadre de la présente invention.

## Revendications

1. Composition cosmétique **caractérisée par le fait qu'**elle comprend, dans un milieu cosmétiquement acceptable,
(A) au moins un polyuréthanne associatif non ionique correspondant à la formule générale 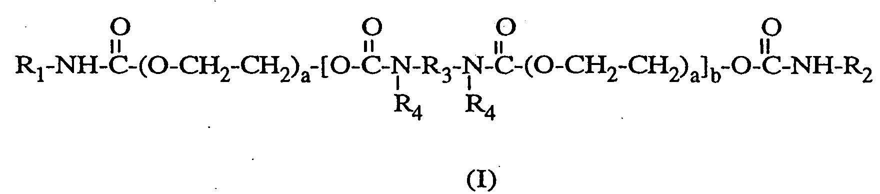 dans laquelle
au moins un des radicaux R₁ et R₂ représente un groupe alkyle supérieur en C₈₋₁₈ et l'autre, le cas échéant, un groupe alkyle inférieur en C₁₋₆,
R₃ représente un radical hydrocarboné en C₄₋₃₆, de préférence en C₆₋₁₀,
R₄ représente un atome d'hydrogène ou un radical alkyle en C₁₋₆, de préférence un atome d'hydrogène,
a varie indépendamment de 90 à 600, et
b vaut de 1 à 4, et
(B) au moins un polymère non ionique comportant au moins un motif à chaîne grasse et qui est différent du polymère de formule (1), choisi parmi
(2) les hydroxypropylguars modifiés par des groupes comportant au moins une chaîne grasse en C₈₋₂₂;
(3) les copolymères de vinylpyrrolidone et de monomères hydrophobes à chaîne grasse en C₈₋₂₂;
(4) les copolymères de méthacrylates ou d'acrylates d'alkyle en C₁₋₆ et de monomères amphiphiles comportant au moins une chaîne grasse en C₈₋₂₂;
(6) les polyuréthannes comportant au moins un motif à chaîne grasse différents des polyuréthannes de formule (I).

2. Composition cosmétique selon la revendication 1, **caractérisée en ce que** le composant (A) est un polyuréthanne associatif non ionique dans lequel un seul des radicaux R₁ et R₂ en α―Ω représente un groupe alkyle supérieur et l'autre un groupe alkyle inférieur.

3. Composition cosmétique selon la revendication 2, **caractérisée en ce que** le composant (A) est un polyuréthanne associatif non ionique dans lequel en moyenne un des radicaux R₁ et R₂ représente un groupe octadécyle et l'autre un groupe méthyle.

4. Composition cosmétique selon l'une quelconque des revendications 1 à 3, **caractérisée par le fait que** le composant (A) se présente sous forme d'une solution ou suspension dans l'eau contenant également de l'amidon soluble modifié par voie chimique, enzymatique ou microbiologique.

5. Composition selon l'une quelconque des revendications 1 à 4, **caractérisée en ce qu'**elle contient le composant (A) à raison de 0,1 à 10 % en poids, et de préférence à raison de 0,5 à 5 % en poids, exprimé en matière active rapportée au poids total de la composition.

6. Composition selon l'une quelconque des revendications 1 à 5, **caractérisée en ce qu'**elle contient le composant (B) à raison de 0,01 à 10 % en poids, de préférence à raison de 0,1 à 5 % en poids de matière active par rapport au poids total de la composition.

7. Composition selon l'une quelconque des revendications 1 à 6 **caractérisée par le fait que** le rapport en poids dudit polyuréthanne associatif non ionique de formule (I) audit polymère non ionique comportant au moins un motif à chaîne grasse est compris dans l'intervalle allant de 90/10 à 10/90.

8. Composition selon l'une quelconque des revendications 1 à 7, **caractérisée par le fait qu'**elle se présente sous forme d'un gel de coiffage ou d'un gel de soin capillaire non rincé.

9. Utilisation de l'association d'un polyuréthanne associatif non ionique de formule (I) et d'un polymère non ionique comportant au moins un motif monomère à chaîne grasse choisi parmi
(2) les hydroxypropylguars modifiés par des groupes comportant au moins une chaîne grasse en C₈₋₂₂;
(3) les copolymères de vinylpyrrolidone et de monomères hydrophobes à chaîne grasse en C₈₋₂₂;
(4) les copolymères de méthacrylates ou d'acrylates d'alkyle en C₁₋₆ et de monomères amphiphiles comportant au moins une chaîne grasse en C₈₋₂₂;
(6) les polyuréthannes comportant au moins un motif à chaîne grasse différents des polyuréthannes de formule (I).
en tant que système épaississant d'une composition cosmétique.

10. Procédé de traitement cosmétique des cheveux, **caractérisé par le fait que** l'on applique sur les cheveux une composition définie selon l'une quelconque des revendications 1 à 9 et en ce que l'on sèche les cheveux ainsi traités sans les rincer.
